# EUROPEAN PATENT SPECIFICATION

(11) **EP 2 257 530 B1**
(45) Date of publication and mention of the grant of the patent: **12.10.2011**
(21) Application number: 09713907.5
(22) Date of filing: 12.02.2009
(51) Int. Cl.: C07D 213/82, C07D 231/14

(54) **PROCESS FOR PREPARING SUBSTITUTED BIPHENYLANILIDES**
VERFAHREN ZUR HERSTELLUNG SUBSTITUIERTER BIPHENYLANILIDE
PROCÉDÉ DE PRÉPARATION DE BIPHÉNYLANILIDES SUBSTITUÉS

(30) Priority: 25.02.2008 EP 08151892
(43) Date of publication of application: 08.12.2010
(73) Proprietor: Bayer CropScience AG, 40789 Monheim (DE)
(72) Inventor: DOCKNER, Michael, 50674 Köln (DE); RIECK, Heiko, 51399 Burscheid (DE)
(86) International application number: PCT/EP2009/000986
(87) International publication number: WO 2009/106234

(56) References cited:
- WO-A-03/070705
- WO-A-2006/092429
- WO-A-2007/138089

## Description

The present invention relates to a process for preparing substituted biphenylanilides of the formula I 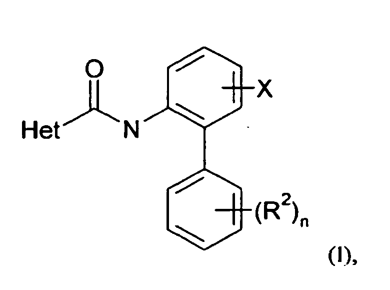 in which the substituents are defined as follows:
Het is a heterocyclyl-residue selected from 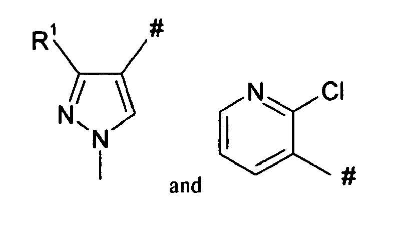 wherein "#" indicates the substituted position of the residue;
X is hydrogen fluorine or chlorine;
R¹ is C₁-C₆-haloalkyl,;
R² is cyano, nitro, halogen, C₁-C₆-alkyl, C₁-C₆-alkenyl, C₁-C₆-alkynyl, C₁-C₆-alkoxy, C₁-C₆-haloalkyl, (C₁-C₆-alkyl)carbonyl or phenyl;
n is 1, 2 or 3, where in case that n is 2 or 3, the R² radicals may also be different, which comprises reacting a compound of formula II 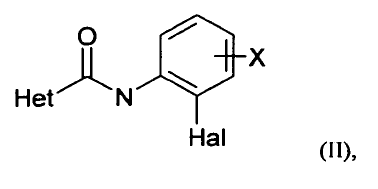 in which Hal is halogen and X and Het are as defined above, in the presence of a base and of a palladium catalyst selected from the group of: a) palladium-triarylphosphine or - trialkylphosphine complex with palladium in the zero oxidation state, b) salt of palladium in the presence of triarylphospine or trialkylphosphine as a complex ligand or c) metallic palladium, optionally applied to support, in the presence of triarylphosphine or trialkylphosphine, in a solvent, with a diphenylborinic acid according to formula (III) 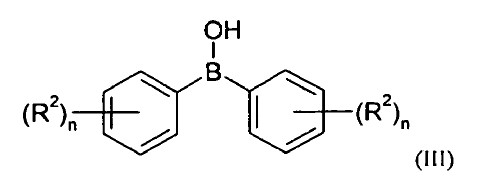 in which R² and n are as defined above, where the triarylphosphines or trialkylphosphines used may be substituted.

Tetrahedron Lett. 32, page 2273 (1991) states that the coupling reaction between phenylboronic acid and chlorobenzene with use of the [1,4-bis(diphenylphosphine)- butane]palladium(II) dichloride catalyst proceeds with a yield of only 28%.

EP-A 0 888 261 discloses a process for preparing nitrobiphenyls by reacting chloronitrobenzenes with a phenylboronic acid in the presence of a palladium catalyst and of a base. In this process, a very high catalyst concentration is necessary.

WO 03/070705 discloses a process for the preparation of biphenylpyrazolecarboxanilides, characterized in that phenylhalopyrazolecarboxanilides are reacted with monophenylboronic acid derivatives in the presence of a catalyst, if appropriate in the presence of an acid binder and if appropriate in the presence of a diluent. Examples of the catalyst to be employed include palladium salts or complexes.

WO 2006/092429 and WO 2007/138089 each pertain to a process for preparing substituted biphenyls by coupling substituted diphenylborinic acids with halo- or dihaloarylcompounds in presence of a palladium catalyst.

It was therefore an object of the present invention to provide an economically viable process which can be implemented on the industrial scale for regioselectively preparing substituted biphenylanilides, which works with a reduced palladium catalyst concentration.

Accordingly, the process defined at the outset has been found.

The diphenylborinic acid (III) is obtained by reaction of optionally substituted phenylmagnesium chloride V with trialkyl borate, preferably trimethyl borate, in tetrahydrofuran as a solvent according to WO 2007/138089 as described by scheme 1.

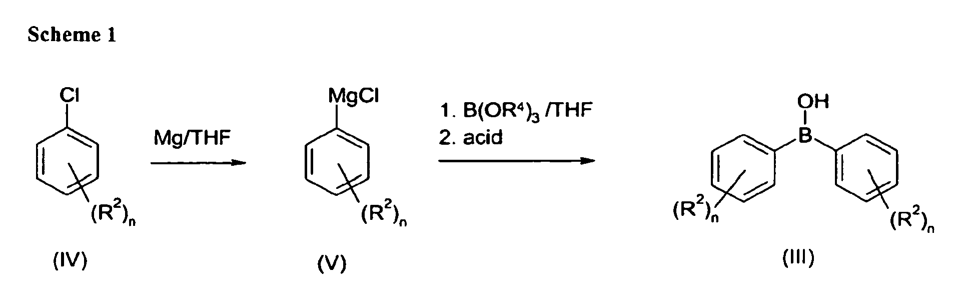

R⁴ is C₁-C₄-alkyl, preferably methyl.

Essential for a high yield of diphenylborinic acid (III) is the use of only 0.7 eq. of trialkyl borate based on the substituted chlorobenzene (IV) used. Use of 1.1 eq, of trialkyl borate gives rise to phenylboronic acid as described in EP-A 0 888 261.

The reaction temperature in this process stage is from 20 to 60°C, preferably from 40 to 50°C.

The substituted biphenyls prepared by the present process have the following preferred substituents, in each case both individually and in combination.
Het is a heterocyclyl-residue selected from 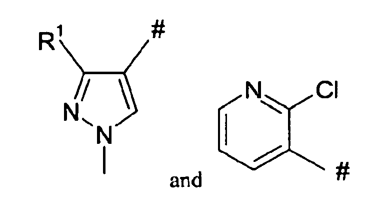 wherein "#" indicates the substituted position of the residue;
R¹ is trifluoromethyl or difluoromethyl, more preferably difluoromethyl;
R² is cyano, nitro, fluorine, chlorine, bromine, methyl, ethyl, propyl, butyl, allyl, propargyl, methoxy, ethoxy, trifluoromethyl or phenyl, more preferably fluorine, chlorine, methyl or methoxy, most preferably fluorine or chlorine;
n is 1 or 2, preferably 2.

The subsequent homogeneously catalyzed Suzuki biaryl cross-coupling is carried out according to scheme 2.

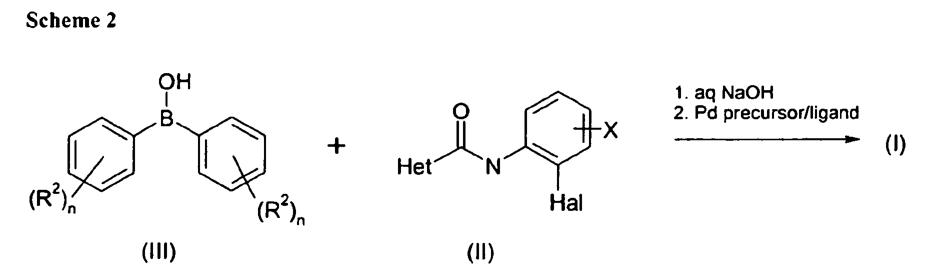

Preference is given to starting from diphenylborinic acids of the formula (III) in which R2 and n are as defined above.

Further preferred starting materials are diphenylborinic acids (III) in which n is 1 or 2, in particular 2. Particularly preferred are diphenylborinic acids (III) which are substituted in the 3- and 4-position or in 4-position only.

Very particular preference is given to di(2,3-difluorophenyl)borinic acid, di(3,4-difluorophenyl)borinic acid, di(2,3-dichlorophenyl)borinic acid and in particular di(3,4-dichlorophenyl)borinic acid and (4-chlorophenyl)borinic acid as the starting compound (III).

Preference is given to starting from the following compounds (II):
N-(2-chloro-4-fluorophenyl)-3-(difluoromethyl)-1-methyl-1H-pyrazole-4-carboxamide, N-(2-bromo-4-fluorophenyl)-3-(difluoromethyl)-1-methyl-1H-pyrazole-4-carboxamide, N-(2-chloro-4-fluorophenyl)-3-(trifluoromethyl)-1-methyl-1H-pyrazole-4-carboxamide, N-(2-bromo-4-fluorophenyl)-3-(trifluoromethyl)-1-methyl-1H-pyrazole-4-carboxamide, N-(2-chloro-6-fluorophenyl)-3-(difluoromethyl)-1-methyl-1H-pyrazole-4-carboxamide, N-(2-bromo-6-fluorophenyl)-3-(difluoromethyl)-1-methyl-1H-pyrazole-4-carboxamide, N-(2-chloro-6-fluorophenyl)-3-(trifluoromethyl)-1-methyl-1H-pyrazole-4-carboxamide, N-(2-bromo-6-fluorophenyl)-3-(trifluoromethyl)-1-methyl-1H-pyrazole-4-carboxamide,N-(2-chlorophenyl)-3-(difluoromethyl)-1-methyl-1H-pyrazole-4-carboxamide, N-(2-bromophenyl)-3-(difluoromethyl)-1-methyl-1H-pyrazole-4-carboxamide, N-(2-chlorophenyl)-3-(trifluoromethyl)-1-methyl-1H-pyrazole-4-carboxamide, N-(2-bromophenyl)-3-(trifluoromethyl)-1-methyl-1H-pyrazole-4-carboxamide, 2-chloro-N-(2-chlorophenyl)pyridine-3-carboxamide, 2-chloro-N-(2-bromophenyl)pyridine-3-carboxamide particularly preferred are N-(2-chloro-4-fluorophenyl)-3-(difluoromethyl)-1-methyl-1H-pyrazole-4-carboxamide, N-(2-bromo-4-fluorophenyl)-3-(difluoromethyl)-1-methyl-1H-pyrazole-4-carboxamide, 2-chloro-N-(2-chlorophenyl)pyridine-3-carboxamide and 2-chloro-N-(2-bromophenyl)pyridine-3-carboxamide.

Compounds according to formula (II) can be prepared as described in WO 03/070705. Using, for example, 1-methyl-3-(trifluoromethyl)-1H-pyrazole-4-carbonyl chloride and 2-bromo-4-fluoroaniline as starting materials and a base as illustrated by the following equation:

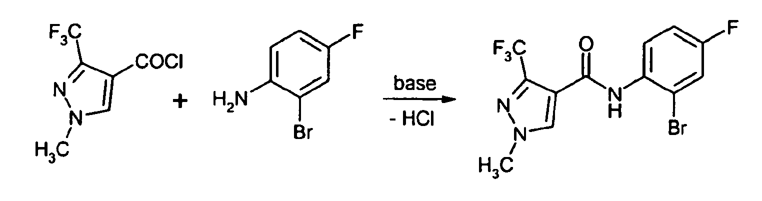

The compound (II) is used, based on the diphenylborinic acids (III) (diphenylborinic acid equivalents), normally in an equimolar amount, preferably with an up to 20 percent excess, in particular with an up to 50 percent excess. The bases used may be organic bases, for example tertiary amines. Preference is given to using, for example, triethylamine or dimethylcyclohexylamine. The bases used are preferably alkali metal hydroxides, alkaline earth metal hydroxides, alkali metal carbonates, alkaline earth metal carbonates, alkali metal hydrogencarbonates, alkali metal acetates, alkaline earth metal acetates, alkali metal alkoxides and alkaline earth metal alkoxides, in a mixture and in particular individually. Particularly preferred bases are alkali metal hydroxides, alkaline earth metal hydroxides, alkali metal carbonates, alkaline earth metal carbonates and alkali metal hydrogencarbonates. Especially preferred bases are alkali metal hydroxides, e.g. sodium hydroxide and potassium hydroxide, and also alkali metal carbonates and alkali metal hydrogencarbonates, e.g. lithium carbonate, sodium carbonate and potassium carbonate. The base is used in the process according to the invention preferably with a fraction of from 100 to 500 mol%, more preferably from 150 to 400 mol%, based on the amount of diphenylborinic acid (III). Suitable palladium catalysts are palladium-ligand complexes with palladium in the zero oxidation state, salts of palladium in the presence of complex ligands, or metallic palladium optionally applied to support, preferably in the presence of complex ligands. Suitable complex ligands are uncharged ligands such as triarylphosphines and trialkylphosphines, which may optionally be substituted in the aryl rings, such as triphenylphosphine (TPP), di-1-adamantyl-n-butylphosphine, tri-tert-butylphosphine (TtBP) or 2-(dicyclohexylphosphino)-biphenyl.

Furthermore, the literature has also described further particularly reactive complex ligands from other structural classes, including 1,3-bis(2,6-diisopropylphenyl)-4,5-H2- imidazolium chloride (cf., for example, G. A. Grasa et al. Organometallics 2002, 21, 2866) and tris(2,4-di-tert-butylphenyl) phosphite (cf. A. Zapf et al., Chem. Eur. J. 2000, 6, 1830).

The reactivity of the complex ligands can be enhanced by adding a quaternary ammonium salt such as tetra-n-butylammonium bromide (TBAB) (cf., for example, D. Zim et al., Tetrahedron Lett. 2000, 41 , 8199). If required, the water solubility of the palladium complexes can be improved by various substituents such as sulfonic acid or sulfonate salt groups, carboxylic acid or carboxylate salt groups, phosphonic acid, phosphonium or phosphonate salt groups, peralkylammonium, hydroxyl and polyether groups. Among the palladium-ligand complexes with palladium in the 0 oxidation state, preference is given to using tetrakis(triphenylphosphine)palladium and additionally tetrakis[tri(o-tolyl)phosphine]palladium. In the salts of palladium which are used in the presence of complex ligands, the palladium is normally present in the two positive oxidation state. Preference is given to using palladium chloride, palladium acetate, palladium acetylacetonate (Pd(acac)₂) or bisacetonitrilepalladium chloride. Particular preference is given to using palladium bisacetylacetonate (Pd(acac)₂).

In general, from 1 to 60, preferably from I to 25, equivalents of the aforementioned complex ligands, in particular triphenylphosphine and, are combined with one equivalent of the palladium salt.

In a most preferred embodiment of the invention palladium bisacetylacetonate and tri-tert-butylphosphine are used in equimolar amounts

EP-A 0 888 261 describes the use of from 2 to 6 equivalents of triphenylphosphine per equivalent of the palladium catalyst. The use of high ligand excesses is generally viewed in the literature as disadvantageous, since this is expected to result in inactivation of the catalytically active complex (cf., for example, J. Hassan et al., Chem. Rev. 2002, 102, 1359). It was thus surprising that this high triphenylphosphine use in combination with the low catalyst use led to an increase in the overall yield of the process of the present invention and accordingly to an improvement in the economic viability. Metallic palladium is used preferably in pulverized form or on a support material, for example in the form of palladium on activated carbon, palladium on alumina, palladium on barium carbonate, palladium on barium sulfate, palladium on calcium carbonate, palladium aluminosilicates such as montmorillonite, palladium on SiO₂ and palladium on calcium carbonate, in each case with a palladium content of from 0.5 to 12% by weight. In addition to palladium and the support material, these catalyst may comprise further dopants, for example lead.

When metallic palladium optionally applied to support is used, particular preference is given to also using the aforementioned complex ligands, in particular to the use of palladium on activated carbon in the presence of triphenylphosphine as a complex ligand, where the phenyl groups in the triphenylphosphine are preferably substituted by a total of from one to three sulfonate groups. In the process according to the invention, the palladium catalyst is used with a low fraction of from 0.001 to 1.0 mol%, preferably from 0.005 to 0.5 mol% or from 0.01 to 0.5 mol% and in particular from 0.005 to 0.05 mol%, based on the amount of compound (II)

The low use of a palladium salt in combination with a high use of a complex ligand constitutes a significant cost advantage of this process over the prior art processes.

The process according to the invention may be carried out in a biphasic system composed of aqueous phase and solid phase, i.e. the catalyst. In that case, the aqueous phase may also comprise a water-soluble organic solvent in addition to water.

Organic solvents suitable for the process according to the invention are ethers such as dimethoxyethane, diethylene glycol dimethyl ether, tetrahydrofuran, dioxane and tert- butyl methyl ether, hydrocarbons such as n-hexane, n-heptane, cyclohexane, benzene, toluene and xylene, alcohols such as methanol, ethanol, 1-propanol, 2-propanol, ethylene glycol, 1-butanol, 2-butanol and tert.-butanol, ketones such as acetone, ethyl methyl ketone and isobutyl methyl ketone, amides such as dimethylformamide, dimethylacetamide and N-methylpyrrolidone, in each case individually or in a mixture.

Preferred solvents are ethers such as dimethoxyethane, tetrahydrofuran and dioxane, hydrocarbons such as cyclohexane, toluene and xylene, alcohols such as ethanol, 1- propanol, 2-propanol, 1-butanol and tert.-butanol, in each case individually or in a mixture. In a particularly preferred variant of the process according to the invention, water, one or more water-insoluble and one or more water-soluble solvents are used, for example mixtures of water and dioxane, or water and tetrahydrofuran, or water, dioxane and ethanol, or water, tetrahydrofuran and methanol, or water, toluene and tetrahydrofuran, preferably water and tetrahydrofuran, or water, tetrahydrofuran and methanol.

The total amount of solvent is normally from 3000 to 500 g and preferably from 2000 to 700 g, per mole of the compound (II).

Appropriately, the process is carried out by adding the compound (II), the diphenyl- borinic acids (III), the base and the catalytic amount of the palladium catalyst to a mixture of water and one or more inert organic solvents, and stirring at a temperature of from 50°C to 120°C, preferably from 70°C to 110°C, more preferably from 90°C to 100°C, for a period of from 1 to 50 hours, preferably from 2 to 24 hours.

Depending on the solvent and temperature used, a pressure of from 1 bar to 6 bar, preferably from 1 bar to 4 bar, is established. Preference is given to carrying out the reaction in water and tetrahydrofuran. The reaction may be carried out in customary apparatus suitable for such processes. On completion of reaction, palladium catalyst obtained as a solid is removed, for example by filtration, and the crude product is freed from the solvent or the solvents. In the case of products which are not fully water-soluble, water-soluble palladium catalysts or complex ligands are removed fully from the crude product in the separation of the water phase. Subsequently, further purification may be effected by methods which are known to those skilled in the art and are appropriate to the particular product, for example by recrystallization, distillation, sublimation, zone melting, melt crystallization or chromatography.

In a further preferred embodiment of the invention the reaction is carried out in toluene or a mixture of water and toluene, such as a 1/1 mixture (water/toluene). On completion of the reaction product and catalyst can be separated by phase separation.

By the process according to the invention, it is possible to prepare, for example:
2-chloro-N-(4'-chlorobiphenyl-2-yl)pyridine-3-carboxamide, N-(3',4'-dichlorobiphenyl-2-yl)-3-(difluoromethyl)-1-methyl-1H-pyrazole-4-carboxamide, N (3',4' dichloro 5-fluorobiphenyl-2-yl)-3-(difluoromethyl)-1-methyl-1H-pyrazole-4-carboxamide, 3-(trifluoromethyl)-1-methyl-1H-pyrazole-4-carboxamide, N-(3',4'-dichloro-5-fluorobiphenyl-2-yl)-3-(trifluoromethyl)-1-methyl-1H-pyrazole-4-carboxamide.

The process according to the invention affords the compounds 1 in very high up to quantitative yields at very good purity. The substituted biphenyls obtainable by the process according to the invention are suitable as precursors for fungicidal crop protection active ingredients (cf. WO 03/070705).

### Examples

### Example 1: Synthesis of Bis(3,4-dichlorophenyl)borinic acid

To a dry flask was added tribromoborane in DCM (13 ml, 13 mmol, 1M). This solution was cooled to -62 °C, and bromo(3,4-dichlorophenyl)magnesium (50 ml, 25 mmol, 0.5M in THF) was added dropwise to the cold solution. The reaction mixture was allowed to warm to room temperature and stirred over night. The solvent was removed in vacuo, and the residue was dissolved in DCM and hydrolyzed by the slow addition of 1N HCl. The organic layer was separated and washed with brine, and the solvent was removed in vacuo. The resulting oil was purified by silica gel chromatography using 25% ethyl acetate as the eluent, which afforded the title compound as a solid (3.34 g, 10.4 mmol, 80% yield).

### Example 2: Synthesis of N-(3',4'-dichloro-5-fluorobiphenyl-2-yl)-3-(difluoromethyl)-1-methyl-1H-pyrazole-4-carboxamide (DMI 3-5)

A 0.5 L double jacket reactor is charged with 178 g of a solution of di(3,4-dichlorophenyl)borinic acid (0.2 mol) in a THF/toluene mixture (2/3 w/w) and 13 g water. A 10% aqueous solution of sodium hydroxide (32 g) is added to adjust the pH to 7.4. N-(2-Bromo-4-fluorophenyl)-1-methyl-3-(difluoromethyl)-1H-pyrazole-4-carboxamide (56 g, 0.2 mol) is added and, subsequently, the reaction mixture is deoxygenated and heated to 75-80°C. At this temperature a mixture of 0.06 g (0.2 mmol) palladium acetylacetonate and 0.06g (0.2 mmol) tri-tert.-butylphosphonium tetrafluoroborate dissolved in a 1/1 mixture of water and toluene, is charged. The pH is adjusted to 8.0 - 8.5 and maintained in this range by addition of a 10 % w/w sodium hydroxide solution (consumption 98 g). After the end of reaction, the reaction mixture is cooled to 20°C and the aqueous phase is separated off (195 g). The organic layer (190 g) contains 22 %w/w (HPLC) of N-(3',4'-dichloro-5-fluorobiphenyl-2-yl)-3-(difluoromethyl)-1-methyl-1H-pyrazole-4-carboxamide (50% yield).

## Claims

1. A process for preparing substituted biphenylanilides of the formula I 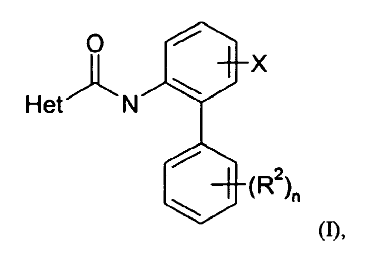
Het is a heterocyclyl-residue selected from 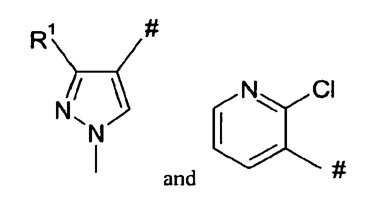 wherein "#" indicates the substituted position of the residue;
X is hydrogen fluorine or chlorine;
R¹ is C₁-C₆-haloalkyl;
R² is cyano, nitro, halogen, C₁-C₆-alkyl, C₁-C₆-alkenyl, C₁-C₆-alkynyl, C₁-C₆-alkoxy, C₁-C₆-haloalkyl, (C₁-C₆-alkyl)carbonyl or phenyl;
R³ is C₁-C₄-alkyl, C₁-C₄-alkenyl or C₁-C₄-alkynyl;
n is 1, 2 or 3, where in case that n is 2 or 3, the R² radicals may also be different,
which comprises reacting a compound of formula II 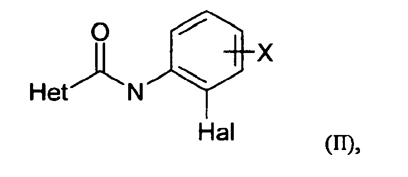 in which Hal is halogen and X and Het are as defined above, in the presence of a base and of a palladium catalyst selected from the group of:
a) palladium-triarylphosphine or -trialkylphosphine complex with palladium in the zero oxidation state,
b) salt of palladium in the presence of triarylphospine or trialkylphosphine as a complex ligand or
c) metallic palladium, optionally applied to support, in the presence of triarylphosphine or trialkylphosphine,
in a solvent, with a diphenylborinic acid according to formula (III) 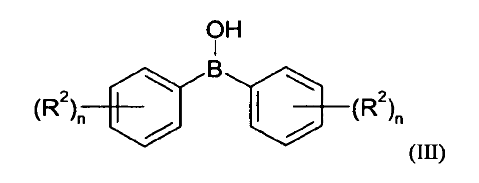 in which R² and n are as defined above, where the triarylphosphines or trialkylphosphines used may be substituted.

2. The process according to claim 1, wherein the compound (II) used is selected from the group consisting of N-(2-chloro-4-fluorophenyl)-3-(difluoromethyl)-1-methyl-1H-pyrazole-4-carboxamide, N-(2-bromo-4-fluorophenyl)-3-(difluoromethyl)-1-methyl-1H-pyrazole-4-carboxamide, 2-chloro-N-(2-chlorophenyl)pyridine-3-carboxamide and 2-chloro-N-(2-bromophenyl)pyridine-3-carboxamide.

3. The process according to claim 1 or 2, wherein the starting compound (III) is a diphenylborinic acid which is substituted in the 3- and 4-position.

4. The process according to claims 1 or 2, wherein a diphenylborinic acid (III) is used which bears fluorine or chlorine in the 3- and 4-positions.

5. The process according to claims 1 or 2, wherein the starting compound (III) is di(3,4-dichlorophenyl)borinic acid.

6. The process according to claims 1 to 5, wherein the palladium catalyst a) according to claim 1 used is tetrakis(triphenylphosphine)palladium or tetrakis(tri-tert.-butylphosphine)palladium.

7. The process according to claims 1 to 5, wherein a palladium catalyst b) according to claim 1 is used.

8. The process according to claims 1 to 5, wherein the palladium catalyst c) according to claim 1 used is metallic palladium on activated carbon in the presence of triphenylphosphine whose phenyl groups are substituted by a total of from 1 to 3 sulfonate groups.

9. The process as claimed in claim 7, wherein the salt of the palladium catalyst b) used is palladium chloride, palladium acetate, palladium bisacetylacetonate (Pd(acac)₂) or bisacetonitrilepalladium chloride.

10. The process according to claim 7, wherein a palladium catalyst b) is used for which from 6 to 60 equivalents of triphenylphosphine are used per equivalent of the palladium salt.

11. The process according to claim 1 , wherein from 0.001 to 1.0 mol% of the palladium catalyst is used, based on the amount of compound (II).

12. The process according to claim 1, wherein the reaction is carried out at a temperature of from 50 to 120°C.

13. The process according to claim 1, wherein the reaction is carried out in a mixture of water and an organic solvent.

14. The process according to claim 13, wherein the organic solvent used is an ether.

15. The process according to claim 1, wherein the reactions are carried out at a pressure of from 1 to 6 bar.

16. The process according to claim 1, wherein compound (II) is N-(2-chloro-4-fluorophenyl)-3-(difluoromethyl)-1-methyl-1H-pyrazole-4-carboxamide and compound (III) is di(3,4-dichlorophenyl)borinic acid.

17. The process according to claim 1, wherein compound (II) is N-(2-chlorophenyl)-3-(difluoromethyl)-1-methyl-1H-pyrazole-4-carboxamide and compound (III) is di(3,4-dichlorophenyl)borinic acid.

18. The process according to claim 1, wherein compound (II) is 2-chloro-N-(2-bromophenyl)pyridine-3-carboxamide and compound (III) is (4-chlorophenyl)borinic acid.

## Patentansprüche

1. Verfahren für die Herstellung von substituierten Biphenylaniliden der Formel 1: 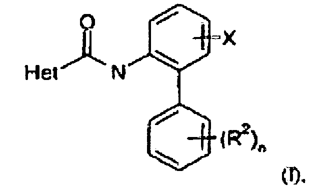
Het ist ein Heterocyclylrest, gewählt aus 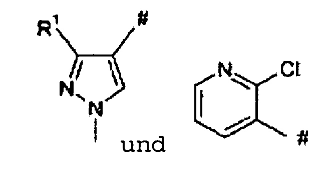 worin "#" die substituierte Position des Rests angibt;
X ist Fluorwasserstoff oder Chlor;
R¹ ist C₁-C₆-Halogenalkyl;
R² ist Cyano, Nitro, Halogen, C₁-C₆-Alkyl, C₁-C₆-Alkenyl, C₁-C₆-Alkinyl, C₁-C₆-Alkoxy, C₁-C₆-Halogenalkyl, (C₁-C₆-Alkyl)-carbonyl oder -phenyl;
R³ ist C₁-C₄-Alkyl, C₁-C₄-Alkenyl oder C₁-C₄-Alkinyl;
n ist 1, 2 oder 3, wobei in dem Fall, dass n 2
oder 3 ist, die R²-Reste ebenfalls verschieden sein können,
umfassend das Umsetzen einer Verbindung der Formel II 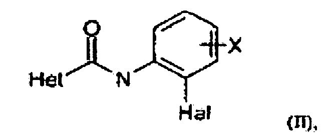 in der Hal ein Halogen ist und X und Het wie oben definiert sind, in Gegenwart einer Base und eines Palladiumkatalysators, gewählt aus der Gruppe von:
a) Palladium-Triarylphosphin oder - Trialkylphosphinkomplex mit Palladium im Oxidationszustand null;
b) Salz von Palladium in Gegenwart von Triarylphosphin oder Trialkylphosphin als einem Komplexliganden, oder
c) metallischem Palladium, gegebenenfalls aufgebracht auf einen Träger, in Gegenwart von Triarylphosphin oder Trialkylphosphin,
in einem Lösungsmittel mit einer Diphenylborinsäure laut Formel (III) 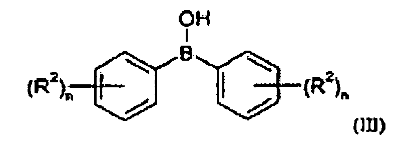 in der R² und n wie oben definiert sind, wobei die verwendeten Triarylphosphine oder Trialkylphosphine substituiert sein können.

2. Verfahren gemäß Anspruch 1, wobei die verwendete Verbindung (II) aus der Gruppe gewählt ist, die aus N-(2-Chlor-4-fluorphenyl)-3-(difluormethyl)-1-methyl-1H-pyrazol-4-carboxamid, N-(2-Brom-4-fluorphenyl)-3-(difluormethyl)-1-methyl-1H-pyrazol-4-carboxamid, 2-Chlor-N-(2-chlorphenyl)-pyridin-3-carboxamid und 2-Chlor-N-(2-bromphenyl)pyridin-3-carboxamid besteht.

3. Verfahren gemäß Anspruch 1 oder 2, wobei die Ausgangsverbindung (III) eine Diphenylborinsäure ist, die an der 3- und 4-Position substituiert ist.

4. Verfahren gemäß Anspruch 1 oder 2, wobei eine Diphenylborinsäure (III) verwendet wird, die Fluor oder Chlor an der 3- und 4-Position trägt.

5. Verfahren gemäß Anspruch 1 oder 2, wobei die Ausgangsverbindung (III) Di(3,4-dichlorphenyl)borinsäure ist.

6. Verfahren gemäß den Ansprüchen 1 bis 5, wobei der verwendete Palladiumkatalysator a) gemäß Anspruch 1 Tetrakis(triphenylphosphin)palladium oder Tetrakis(tri-tert-butylphosphin)palladium ist.

7. Verfahren gemäß den Ansprüchen 1 bis 5, wobei ein Palladiumkatalysator b) gemäß Anspruch 1 verwendet wird.

8. Verfahren gemäß den Ansprüchen 1 bis 5, wobei der verwendete Palladiumkatalysator c) gemäß Anspruch 1 metallisches Palladium auf Aktivkohle in Gegenwart von Triphenylphosphin ist, dessen Phenylgruppen durch insgesamt 1 bis 3 Sulfonatgruppen substituiert sind.

9. Verfahren gemäß Anspruch 7, wobei das Salz des verwendeten Palladiumkatalysators b) Palladiumchlorid, Palladiumacetat, Palladium-bis-acetylacetonat (Pd(acac)₂) oder Bisacetonitrilpalladiumchlorid ist.

10. Verfahren gemäß Anspruch 7, wobei ein Palladiumkatalysator b) verwendet wird, für den 6 bis 60 Äquivalente Triphenylphosphin pro Äquivalent des Palladiumsalzes verwendet werden.

11. Verfahren gemäß Anspruch 1, wobei 0,001 bis 1,0 Mol-% des Palladiumkatalysators, bezogen auf die Menge der Verbindung (II), verwendet werden.

12. Verfahren gemäß Anspruch 1, wobei die Reaktion bei einer Temperatur von 50 bis 120°C durchgeführt wird.

13. Verfahren gemäß Anspruch 1, wobei die Reaktion in einer Mischung von Wasser und einem organischem Lösungsmittel durchgeführt wird.

14. Verfahren gemäß Anspruch 13, wobei das verwendete organische Lösungsmittel ein Ether ist.

15. Verfahren gemäß Anspruch 1, wobei die Reaktionen bei einem Druck von 1 bis 6 bar durchgeführt werden.

16. Verfahren gemäß Anspruch 1, wobei die Verbindung (II) N-(2-Chlor-4-fluorphenyl)-3-(difluormethyl)-1-methyl-1H-pyrazol-4-carboxamid ist und die Verbindung (III) Di(3,4-dichlorphenyl)borinsäure ist.

17. Verfahren gemäß Anspruch 1, wobei die Verbindung (II) N-(2-Chlorphenyl)-3-(difluormethyl)-1-methyl-1H-pyrazol-4-carboxamid ist und die Verbindung (III) Di(3,4-dichlorphenyl)borinsäure ist.

18. Verfahren gemäß Anspruch 1, wobei die Verbindung (II) 2-Chlor-N-(2-bromphenyl)pyridin-3-carboxamid ist und die Verbindung (III) 4-Chlorphenyl)-borinsäure ist.

## Revendications

1. Procédé de préparation de biphénylanilides substitués de formule I 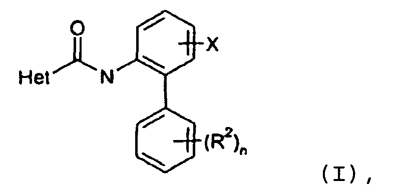 dans laquelle
Het est un résidu hétérocyclyle choisi parmi 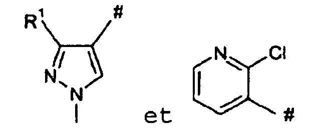 dans lesquels « # » indique la position substituée du résidu ;
X est un hydrogène, un fluor ou un chlore ;
R¹ est un groupe halogénoalkyle en C₁-C₆ ;
R² est un groupe cyano, nitro, halogéno, alkyle en C₁-C₆, alcényle en C₁-C₆, alcynyle en C₁-C₆, alcoxy en C₁-C₆, halogénoalkyle en C₁-C₆, (alkyl en C₁-C₆) carbonyle ou phényle ;
R³ est un groupe alkyle en C₁-C_{4,} alcényle en C₁-C₄ ou alcynyle en C₁ ;
n vaut 1, 2 ou 3, où dans le cas où n vaut 2 ou 3, les radicaux R² peuvent également être différents,
qui comprend la réaction d'un composé de formule II 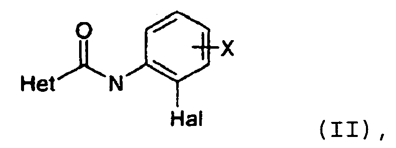 dans laquelle Hal est un halogène et X et Het sont tels que définis ci-dessus, en présence d'une base et d'un catalyseur au palladium choisi dans le groupe constitué par :
a) un complexe palladium-triarylphosphine ou -trialkylphosphine avec du palladium à l'état d'oxydation zéro,
b) un sel de palladium en présence de triarylphosphine ou de trialkylphosphine comme ligand du complexe, ou
c) du palladium métallique, éventuellement appliqué à un support, en présence de triarylphosphine ou de trialkylphosphine,
dans un solvant, avec un acide diphénylborinique répondant à la formule (III) 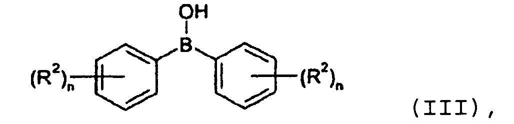 dans laquelle R² et n sont tels que définis ci-dessus, où les triarylphosphines ou trialkylphosphines utilisées peuvent être substituées.

2. Procédé selon la revendication 1, dans lequel le composé (II) utilisé est choisi dans le groupe constitué par le N-(2-chloro-4-fluorophényl)-3-(difluorométhyl)-1-méthyl-1H-pyrazole-4-carboxamide, le N-(2-bromo-4-fluorophényl)-3-(difluorométhyl)-1-méthyl-1H-pyrazole-4-carboxamide, le 2-chloro-N-(2-chlorophényl)pyridine-3-carboxamide et le 2-chloro-N-(2-bromophényl)pyridine-3-carboxamide.

3. Procédé selon la revendication 1 ou 2, dans lequel le composé de départ (III) est un acide diphénylborinique qui est substitué aux positions 3 et 4.

4. Procédé selon la revendication 1 ou 2, dans lequel est utilisé un acide diphénylborinique (III) qui porte du fluor ou du chlore aux positions 3 et 4.

5. Procédé selon la revendication 1 ou 2, dans lequel le composé de départ (III) est l'acide di(3,4-dichlorophényl)borinique.

6. Procédé selon les revendications 1 à 5, dans lequel le catalyseur au palladium a) selon la revendication 1 utilisé est le tétrakis(triphénylphosphine)palladium ou le tétrakis(tri-tert-butylphosphine)palladium.

7. Procédé selon les revendications 1 à 5, dans lequel est utilisé un catalyseur au palladium b) selon la revendication 1.

8. Procédé selon les revendications 1 à 5, dans lequel le catalyseur au palladium c) selon la revendication 1 utilisé est du palladium métallique sur charbon actif en présence de triphénylphosphine dont les groupes phényle sont substitués par un total de 1 à 3 groupes sulfonate.

9. Procédé selon la revendication 7, dans lequel le sel du catalyseur au palladium b) utilisé est le chlorure de palladium, l'acétate de palladium, le bisacétylacétonate de palladium (Pd(acac)₂) ou le chlorure de bisacétonitrilepalladium.

10. Procédé selon la revendication 7, dans lequel est utilisé un catalyseur au palladium b) pour lequel de 6 à 60 équivalents de triphénylphosphine sont employés par équivalent du sel de palladium.

11. Procédé selon la revendication 1, dans lequel de 0,001 à 1,0 % en moles du catalyseur au palladium est utilisé, rapporté à la quantité de composé (II).

12. Procédé selon la revendication 1, dans lequel la réaction est réalisée à une température de 50 à 120 °C.

13. Procédé selon la revendication 1, dans lequel la réaction est réalisée dans un mélange d'eau et d'un solvant organique.

14. Procédé selon la revendication 13, dans lequel le solvant organique utilisé est un éther.

15. Procédé selon la revendication 1, dans lequel les réactions sont réalisées à une pression de 1 à 6 bars.

16. Procédé selon la revendication 1, dans lequel le composé (II) est le N-(2-chloro-4-fluorophényl)-3-(difluorométhyl)-1-méthyl-1H-pyrazole-4-carboxamide et le composé (III) est l'acide di(3,4-dichlorophényl)-borinique.

17. Procédé selon la revendication 1, dans lequel le composé (II) est le N-(2-chlorophényl)-3-(difluorométhyl)-1-méthyl-1H-pyrazole-4-carboxamide et le composé (III) est l'acide di (3,4-dichlorophényl)borinique.

18. Procédé selon la revendication 1, dans lequel le composé (II) est le 2-chloro-N-(2-bromophényl)pyridine-3-carboxamide et le composé (III) est l'acide (4-chlorophényl)borinique.
